(19) Europäisches Patentamt
European Patent Office
Office européen des brevets

(11) Numéro de publication: **0 037 780**
**B1**

(12) # FASCICULE DE BREVET EUROPEEN

(45) Date de publication du fascicule du brevet: **25.05.83**

(51) Int. Cl.³: **C 07 C 93/04**, A 61 K 31/13

(21) Numéro de dépôt: **81400532.8**

(22) Date de dépôt: **02.04.81**

(54) **Alcoxy propanolamines utiles comme médicaments et procédé pour leur préparation.**

(30) Priorité: **04.04.80 FR 8007715**

(43) Date de publication de la demande: **14.10.81 Bulletin 81/41**

(45) Mention de la délivrance du brevet: **25.05.83 Bulletin 83/21**

(84) Etats contractants désignés: **AT BE CH DE FR GB IT LI LU NL SE**

(56) Documents cités:
**FR-A-1 500 464**
**FR-M-7 113**
**US-A-2 712 015**
**US-A-4 196 217**
**CHEMICAL ABSTRACTS, vol. 58, no 10, May 13, 1963, column 10 195 g Columbus, Ohio, US A.F. ISBELL et al.: »Synthesis of solvents for water desalination: amines and ethers«**

(73) Titulaire: **PIERRE FABRE S.A., 125, rue de la Faisanderie, F-75116 Paris (FR)**

(72) Inventeur: **Mouzin, Gilbert, Dr. Chem., 21, rue Sainte-Foy, F-81100 Castres (FR)**
Inventeur: **Cousse, Henri, Dr. Chem., La Foun de los Nobios Chemin de Lastinos, F-81100 Castres (FR)**
Inventeur: **Rieu, Jean-Pierre, Dr. Chem., 10, rue Jean-Mermoz, F-81100 Castres (FR)**

(74) Mandataire: **Corre, Jacques Denis Paul et al, Cabinet Regimbeau 26, Avenue Kléber, F-75116 Paris (FR)**

**EP 0 037 780 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Alcoxy propanolamines utiles comme médicaments et procédé pour leur préparation

La présente invention réalisée au Centre de Recherches Pierre FABRE concerne de nouveaux dérivés d'alcoxy propanolamines possédant des propriétés $\beta$ bloquantes, leur procédé de préparation et leur application en tant que médicaments.

Les substances $\beta$ bloquantes sont des analogues structuraux de la noradrénaline, ils sont susceptibles de se fixer sur les récepteurs $\beta$ et de s'opposer à la fixation des médiateurs chimiques analogues empêchant ainsi la naissance de la réaction biologique.

Tous les bloquants actuellement commercialisés appartiennent à la famille des phényl éthanol amines (PEA) de formule générale:

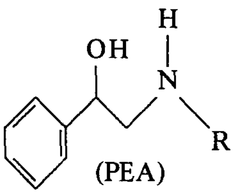

(PEA)

ou à celle des aryloxypropanolamines (A.O.P.A.) de formule générale:

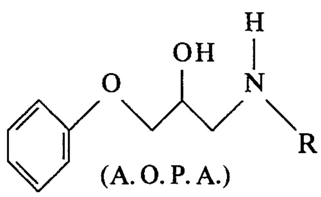

(A. O. P. A.)

GADRET et coll. — Eur. J. Med. Chem. 4, 367 (1978) ont montré par la méthode PCILO corrélée par une étude par diffraction des rayons X, l'importance du noyau aromatique dans ce type de structure.

Le brevet français n° 7 113 M décrit des produits à activité $\beta$-bloquante ayant une structure voisine de celle des AOPA mais dans lesquels le radical phényle est remplacé par un radical acétylénique.

Quant au brevet des Etats-Unis d'Amérique n° 4 196 217, celui-ci décrit des amines hydroxylées présentant une activité bactéricide.

La demanderesse a découvert que certaines alcoxy propanol amines dont la structure présente une différence fondamentale avec les PEA et AOPA, provenant de l'absence de noyau aromatique, exercent de façon surprenante une activité $\beta$ bloquante utile dans le traitement des troubles cardio-vasculaires.

L'invention a notamment pour objet, à titre de médicament, des alcoxy propanolamines de formule générale:

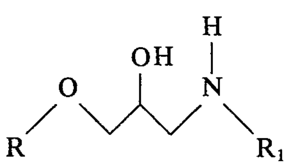

(I)

dans laquelle:

R est un radial alcoyle en $C_1$ à $C_{20}$,
$R_1$ est un radial isopropyle ou terbutyle,

ainsi que les sels des composés de formule I, obtenus par salification avec des acides thérapeutiquement acceptables.

Parmi les radicaux alcoyles mentionnés précédemment dans la définition de R, il faut citer plus particulièrement les radicaux pouvant être linéaires ou ramifiés, par exemple n-butyl, n-hexadécyl, butyl-2, méthyl-2-butyl-1, hexanyl-2.

Par »acides thérapeutiquement acceptables« on entend désigner des acides minéraux tels que l'acide chlorhydrique, phosphorique et sulfurique ou des acides organiques notamment monocarboxyliques ou dicarboxyliques tels que les acides succiniques, tartriques, oxaliques, maléiques et fumariques.

Dans le cas des polyacides, les sels peuvent être les sels correspondant à la neutralisation d'une ou plusieurs des fonctions acides.

Les composés selon la présente invention répondent donc aux formules I' et I'':

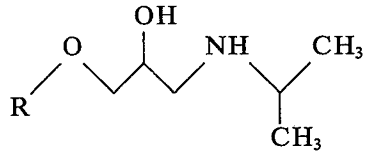

(I')

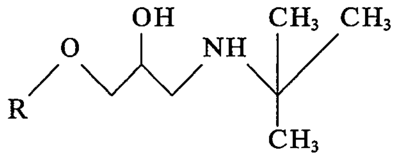

(I'')

dans lesquelles:

R a la même signification que précédemment.

La présente invention concerne également un procédé de préparation des composés de formule I, et en particulier des composés de formule I' et I'', par réaction d'un époxyde de formule II:

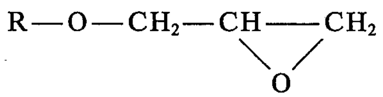

(II)

sur une amine primaire de formule III:

$R_1-NH_2$ (III)

dans un solvant en partculier un alcool tel que le méthanol.

L'époxyde de formule II peut être préparé par action de l'épichlorhydrine sur un alcool de formule IV:

$R-OH$ (IV)

en présence d'une base telle que la soude et d'un catalyseur en particulier un composé d'ammonium quaternaire notamment de formule:

$^{\oplus}N(R_2)_4X^-$

dans laquelle $R_2$ est un radical alcoyle inférieur et $X^-$ est l'anion d'un acide par exemple $HSO_3^-$ ou $Hal^-$; tel que l'hydrogéno-sulfate de tétrabutylammonium.

Les alcools et les amines mis en oeuvre dans ce procédé sont des composés connus de la technique antérieure.

La présente invention concerne également l'utilisation des composés de formule I à titre de médicament, ainsi que des compositions pharmaceutiques contenant ces médicaments.

Les compositions pharmaceutiques selon la présente invention peuvent comporter un ou plusieurs composés de formule I éventuellement en association avec d'autres principes actifs.

Les exemples suivants illustrent la préparation de certains composés selon la présente invention, sans, bien entendu, en limiter la portée.

## Exemple 1

### Préparation de l'isopropylamino-3 butoxy-1 propanol-2

#### A) Préparation du butoxy-1 époxy-2,3 propane

Dans un tricol renfermant un mélange de 150 ml de soude à 50%, 150 ml d'épichlorhydrine et 5,5 g d'hydrogèno-sulfate de tétra butylammonium, on ajoute sous vive agitation 37 ml (30 g — 0,4 mole) de butanol de telle façon que la température reste inférieure à 20°C. (Refroidir par un bain de glace.)

Après addition le mélange est traité par un litre d'eau et extrait trois fois à l'éther. La phase organique est lavée avec une solution de bicarbonate de sodium, puis à l'eau saturée de sel et séchée sur sulfate de sodium.

La phase éthérée est récupérée et évaporée jusqu'à siccité et rectifiée sous vide.

On récupère avec un rendement de 78%, 40,7 g de produit. $Eb_{30}=76-78°C$.

B) Préparation de l'isopropylamino-3 butoxy-1 propanol-2

A une solution de 15 g (0,115 mole) de butoxy-1 époxy-2,3 propane dans 150 ml de méthanol, refroidie sur bain de glace, on ajoute 40 ml (27,4 g — 0,46 mole) d'isopropylamine.

Après une nuit à température ambiante le mélange est évaporé jusqu'à siccité. On récupère après rectification sous vide 17 g de produit (rendement 80%) de formule:

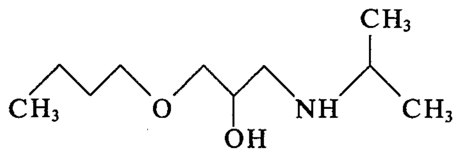

Formule brute: $C_{10}H_{23}NO_2$
Masse moléculaire: 189,3
Huile: incolore
Point ébullition: 70° C (0,01 mm Hg)

$$n_D^{23,3} = 1,4430$$

Chromatographie sur plaque:

— support: gel de silice 60 F 254 Merck
— solvant: chloroforme — méthanol — ammoniaque 80/18/2
— révélation: iode
— Rf: 0,65

Solubilité: soluble à 5% dans l'eau.

Exemple 2

Préparation du tertiobutylamino-3 butoxy-1 propanol-2

A une solution de 14,3 g (0,11 mole) de butoxy-1 époxy 2—3 propane dans 150 ml de méthanol, refroidie sur bain de glace, on ajoute 24,15 g (0,33 mole) de tertiobutylamine. Après cinq heures d'agitation à température ambiante, on porte 1 H 30 à reflux.

L'alcool et l'excès d'amine sont évaporés sous pression réduite et l'huile résiduelle est rectifiée sous vide. On récupère avec un rendement de 75% le produit de formule:

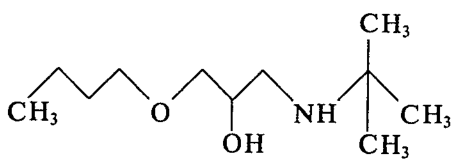

Formule brute: $C_{11}H_{25}NO_2$
Masse moléculaire: 203,32
Cristaux blancs
Point de fusion: 21° C
Point d'ébullition: 76° (0,015 mm Hg) (huile incolore)

$$n_D^{23} = 1,4430$$

Chromatographie sur plaque:

— support: gel de silice 60 F 254 Merck
— solvant: chloroforme — méthanol — ammoniaque 80/18/2
— révélation: iode
— Rf: 0,6

Solubilité: soluble à 1% dans l'eau.

## Exemple 3

Préparation du tertiobutylamino-3 (butyl-2) oxy-1 propanol-2

D'une façon similaire à celle décrite dans l'exemple 1, mais en utilisant le sec-butanol et la tertiobutylamine, on obtient le produit de formule:

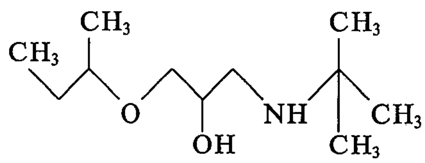

Formule brute: $C_{11}H_{25}NO_2$
Masse moléculaire: 203,3

## Exemple 4

Préparation de l'isopropylamino-3 (méthyl-2 butyl-1) oxy-1 propanol-2

D'une manière similaire à celle décrite dans l'exemple 1, mais en utilisant le méthyl-2 butanol, on obtient le produit de formule:

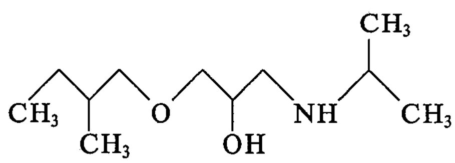

Formule brute: $C_{11}H_{25}NO_2$
Masse moléculaire: 203,3

## Exemple 5

Chlorhydrate de tertiobutylamino-3 hexadecyloxy-1 propanol-2

D'une manière similaire à celle décrite dans l'exemple 1 mais en utilisant l'hexadecanol, la terbutylamine et en salifiant par l'acide chlorhydrique, on obtient le produit de formule:

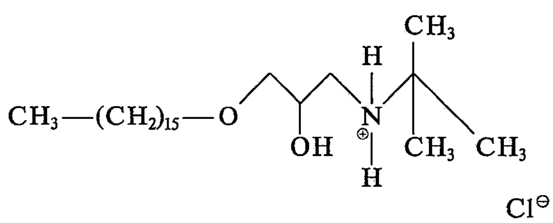

Formule brute: $C_{23}H_{50}ClNO_2$
Masse moléculaire: 408,1

## Exemple 6

Chlorhydrate de tertiobutylamino-3 (Hexanyl-2) oxy-1 propanol-2

D'une façon similaire à celle décrite dans l'exemple 1 mais en utilisant l'hexanol-2, la terbutylamine et en salifiant par l'acide chlorhydrique, on obtient le produit de formule:

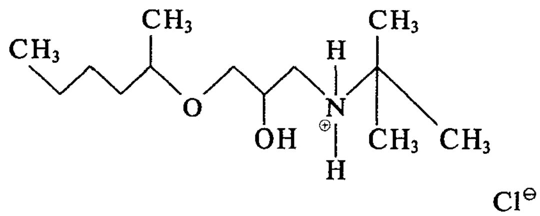

Formule brute: $C_{13}H_{30}ClNO_2$
Masse moléculaire: 267,8

Des expérimentations ont permis de mettre en évidence les propriétés pharmacologiques des composés selon la présente invention.

A) Toxicologie

Les composés chimiques précédemment décrits ont été soumis à des contrôles de toxicité.

L'étude de la toxicité a été effectuée chez des souris pesant de 20 à 22 grammes.

Les substances ont été administrées par voie intra-veineuse et orale. La $DL_{50}$ est calculée selon la méthode de KARBER — Arch. Expl. Pathol. Pharmacol., 1931, 162, 480.

Par voie intra-veineuse les $DL_{50}$ sont comprises entre 20 et 100 mg/kg; par voie orale les $DL_{50}$ sont comprises entre 700 et 1000 mg/kg.

B) Etude pharmacologique

Les expérimentations pharmacologiques auxquelles ont été soumis les composés chimiques, objet de l'invention, ont permis de mettre en évidence des propriétés $\beta$ bloquantes selon la méthode de DUNLOP O. et SHANK P. — Brit. J. Pharmacol., 1968, 32, 201 à 218. Méthode: Tachycardie isoprénalique chez le chien. Enregistrement simultané de la fréquence cardiaque et de la pression artérielle.

Résultats: Les produits les plus intéressants réduisent significativement la tachycardie isoprénalique à partir de la dose de 0,1 mg/kg par voie intraveineuse. De plus certains composés et plus particulièrement le composé de l'exemple 1 présente une activité $\beta$ bloquante sélective.

C) Applications thérapeutiques

Compte tenu de leurs propriétés pharmacologiques et de leur faible toxicité, ces composés et plus spécialement le composé de l'exemple 1 peuvent être utilisés en thérapeutique dans les traitements de troubles cardiaques comme par exemple l'hypertension, l'arythmie cardiaque et l'angine de poitrine.

En raison de l'effet $\beta$ bloquant sélectif, on peut utiliser avec avantage un des composés et plus spécialement le composé de l'exemple 1, en association avec un bronchodilatateur sympathomimétique comme l'isoprénaline ou l'éphédrine, pour le traitement de l'asthme et d'autres maladies obstructives des voies aériennes, du fait que l'effet sélectif du composé inhibe sensiblement la stimulation cardiaque indésirable du bronchodilatateur sans en gêner son effet thérapeutique.

Ces composés peuvent être utilisés sous la forme de compositions pharmaceutiques dans lesquelles on mélange le composé actif avec des diluants ou véhicules non toxiques pharmaceutiquement acceptables. On peut administrer ces compositions par voie parentérale, intraveineuse ou par voie orale ou rectale.

Les compositions peuvent être par exemple sous la forme de comprimés, de capsules, de suppositoires, de solutions aqueuses ou huileuses, de suspensions aqueuses ou huileuses, d'émulsions, de poudres dispersables ou de solutions ou suspensions aqueuses ou huileuses injectables.

Ces compositions peuvent en particulier se présenter sous des formes galéniques modifiant leur biodisponibilité, soit en favorisant une action rapide, soit au contraire en procurant une action retard.

Les posologies utilisables dépendent du type de composés utilisés, de la voie d'administration et du type d'affection à traiter et peuvent être déterminées par des tests connus.

**Revendications**

1. Composés chimiques de formule I:

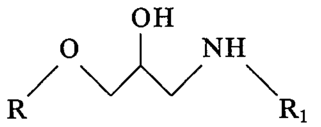

(I)

dans laquelle R représente un radical alcoyle en $C_1$ à $C_{20}$, $R_1$ représente un radical isopropyle ou terbutyle, ainsi que les sels d'acides pharmaceutiquement acceptables de ces composés.

2. Composés selon la revendication 1, sous forme de sel avec l'acide chlorhydrique, sulfurique, phosphorique, maléique, succinique, tartrique, fumarique et oxalique.

3. Composé selon la revendication 1, l'isopropylamino-3 butoxy-1 propanol-2.

4. Composé selon la revendication 1, le tertiobutylamino-3 butoxy-1 propanol-2.

5. Composé selon la revendication 1, le tertiobutylamino-3 (butyl-2)oxy-1 propanol-2.

6. Composé selon la revendication 1, l'isopropylamino-3 (méthyl-2 butyl-1)oxy-1 propanol-2.

7. Composé selon l'une des revendications 1 et 2, le chlorhydrate de tertiobutylamino-3 hexadécycloxy-1 propanol-2.

8. Composé selon l'une des revendications 1 et 2, le chlorhydrate de tertiobutylamino-3 (hexanyl-2)oxy-1 propanol-2.

9. Procédé de préparation d'un composé selon l'une des revendications 1 à 8, caractérisé en ce que l'on fait réagir l'épichlorhydrine sur un alcool de formule IV:

$$R-OH \qquad (IV)$$

en présence d'une base et d'un catalyseur du type ammonium quaternaire pour obtenir l'époxyde intermédiaire de formule II:

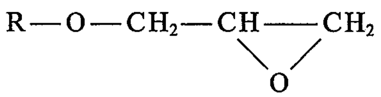

(II)

que l'on fait réagir ensuite sur une amine primaire de formule III:

$$R_1-NH_2 \qquad (III)$$

pour obtenir les dérivés revendiqués de formule I.

10. A titre de médicament nouveau utile en thérapeutique cardio-vasculaire, un composé selon l'une des revendications 1 à 8.

11. Composition pharmaceutique caractérisée en ce qu'elle contient à titre de principe actif au moins un composé selon l'une des revendications 1 à 8.

**Patentansprüche**

1. Chemische Verbindungen mit der Formel I:

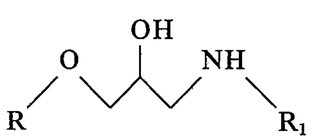

(I)

in welcher R ein Alkylradikal von $C_1$ bis $C_{20}$, $R_1$ ein Isopropyl- oder Terbutylradikal darstellt, sowie die Salze mit pharmazeutisch annehmbaren Säuren dieser Verbindungen.

2. Verbindungen nach Anspruch 1, in Form von Salz mit Salzsäure, Schwefelsäure, Phosphorsäure, Maleinsäure, Bernsteinsäure, Weinsäure, Fumarsäure und Oxalsäure.

3. Verbindung nach Anspruch 1, Isopropylamino-3 butoxy-1 propanol-2.

4. Verbindung nach Anspruch 1, Tertiobutylamino-3 butoxy-1 propanol-2.

5. Verbindung nach Anspruch 1, Tertiobutylamino-3 (butyl-2)oxy-1 propanol-2.

6. Verbindung nach Anspruch 1, Isopropylamino-3 (methyl-2 butyl-1)oxy-1 propanol-2.

7. Verbindung nach einem der Ansprüche 1 und 2, Tertiobutylamino-3 hexadecyloxy-1 propanol-2-chlorhydrat.

8. Verbindung nach einem der Ansprüche 1 und 2, Tertiobutylamino-3 (hexanyl-2)oxy-1 propanol-2-chlorhydrat.

9. Verfahren zur Herstellung einer Verbindung nach einem der Ansprüche 1 bis 8, dadurch gekennzeichnet, daß man Epichlorhydrin auf einen Alkohol der Formel IV

R–OH (IV)

einwirken läßt, in Gegenwart einer Base und eines Katalysators vom Typ einer quartären Ammoniumverbindung, um als Zwischenprodukt ein Epoxyd der Formel II

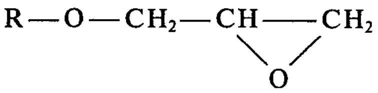 (II)

zu erhalten, das man dann auf ein primäres Amin der Formel III

$R_1-NH_2$ (III)

einwirken läßt, um die beanspruchten Derivate der Formel I zu erhalten.

10. Eine Verbindung nach einem der Ansprüche 1 bis 8, als neues Medikament verwendbar in der Herz-Gefäß-Therapeutik.

11. Pharmazeutische Zusammensetzung, dadurch gekennzeichnet, daß sie als aktives Mittel wenigstens eine Verbindung nach einem der Ansprüche 1 bis 8 enthält.

**Claims**

1. Chemical compounds of formula I:

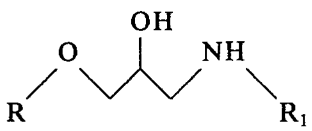 (I)

in which R represents a $C_1$ to $C_{20}$ alkyl radical, $R_1$ represents an isopropyl or tertbutyl radical, as well as the pharmaceutically acceptable acid salts of these compounds.

2. Compounds according to claim 1, in the form of a salt with hydrochloric, sulphuric, phosphoric, maleic, succinic, tartaric, fumaric and oxalic acid.

3. Compound according to claim 1, being 3-isopropylamino-1-butyoxy-2-propanol.

4. Compound according to claim 1, being 3-tertiarybutylamino-1-butoxy-2-propanol.

5. Compound according to claim 1, being 3-tertiarybutylamino-1-(2-butyl)oxy-2-propanol.

6. Compound according to claim 1, being 3-isopropylamino-1-(2-methyl-1-butyl)oxy-2-propanol.

7. Compound according to one of claims 1 and 2, being 3-tertiarybutylamino-1-hexadecyloxy-2-propanol chlorhydrate.

8. Compound according to one of claims 1 and 2, being 3-tertiarybutylamino-1-(2-hexanyl)oxy-2-propanol chlorhydrate.

9. Method of preparing a compound according to one of claims 1 to 8, characterised in that epichlorhydrin is made to react with an alcohol of formula IV:

R–OH (IV)

in the presence of a base and a catalyst of the quaternary ammonium type in order to obtain the intermediate epoxide of formula II:

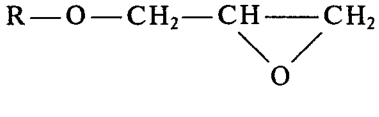 (II)

which is subsequently made to react with a primary amine of formula III:

$R_1-NH_2$ (III)

in order to obtain the claimed derivatives of formula (I).

10. A compound according to one of claims 1 to 8 as a novel medicament used in cardio-vascular therapy.

11. Pharmaceutical composition characterized in that it containes at least one compound according to one of claims 1 to 8 as the active principle.